(19) Europäisches Patentamt
European Patent Office
Office européen des brevets

(11) **EP 0 510 175 B1**

(12) **EUROPEAN PATENT SPECIFICATION**

(45) Date of publication and mention
of the grant of the patent:
**12.06.1996 Bulletin 1996/24**

(51) Int Cl.6: **G01N 21/00, G01N 21/01,
G01N 33/00, G01N 21/77,
G01N 21/64, G01N 33/543**

(21) Application number: **92900955.3**

(22) Date of filing: **08.11.1991**

(86) International application number:
**PCT/US91/08390**

(87) International publication number:
**WO 92/08966 (29.05.1992 Gazette 1992/12)**

(54) **FLUORESCENCE ASSAY APPARATUS**

APPARAT ZUR FLUORESZENZANALYSE

APPAREIL D'ANALYSE PAR FLUORESCENCE

(84) Designated Contracting States:
**DE FR GB**

(30) Priority: **13.11.1990 US 612869**

(43) Date of publication of application:
**28.10.1992 Bulletin 1992/44**

(73) Proprietor: **Block, Myron J.
North Salem New Hampshire 03073 (US)**

(72) Inventors:
• **BLOCK, Myron, J.
North Salem, NH 03073 (US)**

• **LACKIE, Steve, J.
Lexington, MA 02173 (US)**

(74) Representative: **Thomson, Paul Anthony
Potts, Kerr & Co.
15, Hamilton Square
Birkenhead Merseyside L41 6BR (GB)**

(56) References cited:
WO-A-90/15985          US-A- 3 604 927
US-A- 3 975 084        US-A- 4 050 895
US-A- 4 558 014        US-A- 4 582 809
US-A- 4 608 344        US-A- 4 810 658
US-A- 4 909 900

## Description

This invention relates to optical apparatus for effecting fluorescence assays of chemical and biochemical ligands, and more particularly to a system for separating the desired assay information in the output fluorescence from unwanted background fluorescence forming part of that output.

Among the extensive chemical and biochemical systems used for analysis or assays, are optical systems employing the principles of attenuated total internal reflection (ATR) spectroscopy. Particularly useful for immunoassays, such optical systems employ an optical fiber or rod upon a portion (i.e., a sensing zone) of the outer surface of which an antibody, for example, is covalently immobilized, adsorbed or the like. That antibody is selected to be reactive with a ligand such as an antigen to be assayed or tested in a solution. A light beam introduced into one end of the optical fiber will be totally internally reflected in the dense medium of the fiber, and will generate, in the surrounding rarer medium or test solution an electromagnetic waveform, known as the evanescent wave component. The latter, for practical purposes, characteristically effectively extends only a fraction of a wavelength across the interface between the fiber and test solution. This penetration, however, is sufficient to permit substantial optical interaction in the sensing zone between the evanescent wave component and the immobilized antibody with which the antigen in the test solution will complex, and only minimally with any bulk solution in which the antigen may be present. Such optical interaction then permits one to assay the antigen. A number of such systems using total internal reflection spectroscopy for an assay are known and have been described. For example, U.S. Patent No. 4,133,639 discloses a system based on absorption of the evanescent wave by the analyte; and U.S. Patent Nos. 4,321,057 and 4,399,099, both disclose systems that detect changes in the radiation transmitted through the fiber; and U.S. Patent No. 4,447,546, describes a fluorescence immunoassay system.

In the latter system, fluorescent radiation arising in the sensing zone due to excitation by the evanescent wave, tunnels back into the fiber within the total reflection angle and is thus trapped within the fiber. The amplitude of fluorescence increases with the length of the sensing zone along the fiber. However, the optical throughput of the system (determined by the diameter and the numerical aperture of the fiber) remains constant. The total fluorescent signal coming from the entire surface of the sensing zone along the fiber, enhanced by the increase in fluorescent moieties diffusing into the zone during excitation, thus becomes available in a bright spot (i.e., of dimensions set by the cross-section of the fiber), exciting the fiber at its input end through a restricted angle determined by the critical angle of reflection within the fiber. Such signal is easily collected at high efficiency and throughput matched to a small detector.

For example, U.S. Patent No. 4,050,895 discloses exciting a waveguide at its input end through an annular input aperture mask that blocks axial light rays and defines the range of cone angles for light rays propagating through the waveguide. The reference also discloses a second mask positioned at the waveguide exit at the opposite end from the waveguide input, to mask direct light which has not undergone any surface interaction.

As is well known, for excitation radiation initially propagating through an optical fiber of refractive index $n_0$, otherwise surrounded by a material of refractive index $n_1$, the maximum acceptance angle B of input radiation into the fiber can be found from the equation:

$$(1) \qquad NA = n_2 \sin b = (n_0^2 - n_1^2)^{1/2}$$

where $n_2$ is the refractive index of the medium (typically air) through which the radiation is initially propagated so as to be incident upon an end of the fiber, and NA is the so-called numerical aperture of the fiber. The maximum acceptance angle B is then simply defined as:

$$(2) \qquad B = \sin^{-1} NA$$

and B=b when $n_2$=1 (e.g., $n_2$ is for dry air). Thus, the numerical aperture at the sensing zone of a fiber is highest when the fiber core material has a very high index and the medium surrounding the sensing zone has a very low index, or $n_0 >> n_1$. For example, satisfactory sensitivities can be obtained where a transparent fiber (glass, silica, polymer or the like) of ordinary index of refraction is surrounded by an aqueous solution that typically has an index of refraction in the vicinity of 1.33-1.35.

Although known fluorescence immunoassay apparatus of the type discussed above functions satisfactorily, it tends to be relatively expensive to conduct an immunoassay when the optical fiber used with such known apparatus is made from quartz, because of the relatively high cost of quartz optical fibers and particularly where the fiber is discarded following each assay. Also, it tends to be relatively difficult and hence costly to achieve a satisfactory flow cell-fiber interface with quartz fibers.

An obvious solution to these problems is to use an injection-molded synthetic polymer fiber in place of the quartz fiber. Unfortunately, polymers such as polystyrene, which are usable as optical fibers, generally will themselves be excited into emission of broad-band radiation, when exposed to appropriate excitation light. This creates undesired background at least part of which is within the same radiation band as the desired fluorescence generated as a consequence of specific binding of the constituents of the immunoassay complex. As such, it can be extremely difficult to determine that portion of the output radiation that is of interest, i. e., the fluorescence attributable to desired specific binding. To further complicate matters, the internal emissions from the polymeric fibers decays rapidly and inconsistently over the same time scale as the assay. The actual mechanism whereby the polymer generates the undesired background is uncertain, but is believed to be fluorescent, although it is postulated that it may be phosphorescence or even Raman emission, but will generally be deemed fluorescent for purposes of exposition.

This problem of unwanted fluorescence has been overcome in certain fluorescence systems incorporating specific polymeric components. For instance, in microtest well systems in which fluorescent tags are used in one constituent of the immunochemical complex to be assayed, the problem of unwanted fluorescence has been avoided in at least two ways. For example, as disclosed in U.S. Patent No. 4,501,970, a pigment is added to the plastic assay panels used to support the test samples to make the panels opaque. Also, as disclosed in U.S. Patent No. 4,725,388, the sample is read through the open top of the polymeric microtest well so that substantially only the test sample, and not the well, is in the field of view.

These two solutions to the problem of unwanted fluorescence are not practically applicable to fluorescence assay apparatus employing synthetic polymeric optical fibers. Because such fluorescence assay systems are based on the ability of the fiber to transmit light, the addition of a pigment to render the fiber opaque clearly is not a solution to the problem of fiber fluorescence. Similarly, because the detector of the fluorescence assay device must maintain the input/output end of the fiber in its field of view, the solution to the polymer fluorescence problem disclosed in U.S. Patent No. 4,725,388 is also not a viable option.

One object of the present invention is to provide a fiber-optic assay system employing a synthetic polymeric optical fiber as the substrate element on the surface of which is bound one constituent of the assay complex.

Another object of the present invention is to provide a system for determining those portions of the output fluorescence of the optical element of a fluorescence immunoassay system attributable to unwanted background fluorescence and to desired fluorescence from ligands specifically bound to the surface of the element.

Yet another object of the present invention is to provide a method of performing a fluorescence assay using a polymeric optical fiber, notwithstanding internal generation of undesired background fluorescence by the polymeric fiber.

These and other objects are achieved by providing, in an assay system including an elongated optical fiber capable of internally propagating excitation radiation therethrough by total internal reflection, a source of the excitation radiation, and means for directing the apical portion of an input cone of such radiation toward an input or proximal end of the fiber, an assay system comprising means for determining the distribution in angle of fluorescent radiation excited by the excitation radiation and emitted from an end of the fiber, the background portion of the fluorescence being determinable from such distribution. Particularly, such distribution may be determined using means for varying the numerical aperture (NA) adjacent a sensing zone at the surface of the fiber, by alternately controlling the admission of excitation radiation into the fiber so that only selected colinear conical portions of the input cone will propagate along the fiber, and controlling the egress of the fluorescence from the fiber so that only selected colinear conical portions of the fluorescent emission are examined. The means for varying the numerical aperture comprises means for selecting first and second such portions of the input cone, which portions, when introduced into the fiber respectively provide a relatively high numerical aperture and a relatively lower numerical aperture at the active zone or part of the fiber, means also being provided for alternately introducing those conical portions into the input end of the fiber concentrically therewith. Similarly, the output fluorescent radiation is divided, either alternately or concurrently, into first and second portions representing the emissions arising from respective excitation at the sensing zone by the portions of the excitation radiation. It should be understood that the term "colinear conical portion" as used herein is intended to mean that portion of a cone that shares the same axis of revolution as another cone, has an outer nappe that is conical but not necessarily congruent with the nappe of the other cone, and includes annuli thereof.

Typically, the system further includes detector means for detecting respective portions of the fluorescence emitted from, preferably, the input end of the optical fiber and for providing an output signal containing information which varies as a function of the intensity of the detected fluorescence. The system also preferably includes a computer coupled with the detector means for determining from the foregoing information, which portion of the total fluorescence emitted from the fiber is attributable to fluorescence generated as a consequence of the binding of a ligand of interest into the complex on the fiber.

In a preferred embodiment of the invention, the means for varying the numerical aperture comprises an opaque plate mounted for movement with respect to the cone of input radiation between a first position in which the plate constitutes means for selecting a first colinear conical portion (such as an outer annulus) of the cone, and a second position in which the plate constitutes means for selecting a second colinear conical portion (such as the inner core or conical center) of the cone.

Other objects of the invention will in part be obvious and will in part appear hereinafter. The invention accordingly comprises the apparatus possessing the construction, combination of elements, and arrangement of parts which are exemplified in the following detailed disclosure, and the scope of the application of which will be indicated in the claims.

For a fuller understanding of the nature and objects of the present invention, reference should be made to the following detailed description taken in connection with the accompanying drawings wherein:

Fig. 1 is a schematic illustration of a fluorescence assay system embodying the principles of the present invention;

Fig. 2 is a schematic side elevational view of the proximal end of the optical fiber, the objective lens, and one embodiment of the means for varying the NA of the present invention;

Fig. 3 is a plot of an idealized waveform of the output of the detector of an embodiment of the present invention;

Fig. 4 is a schematic front elevational view showing the two positions of a plate of another embodiment of the means for varying the NA;

Fig. 5 is another schematic front elevational view showing the two positions of a plate of yet another embodiment of the means for varying the NA;

Fig. 6 is another schematic front elevational view similar to the embodiment of Fig. 5, of another means for varying the NA positioned in front of and adjacent the proximal end of the fiber;

Fig. 7 is similar to Fig. 1, but includes an alternative embodiment of the detection system for detecting the output fluorescence from the optical fiber;

Fig. 8 is similar to Fig. 7, but illustrates yet another alternative embodiment of the detection system; and

Fig. 9 is a front elevational view of the detector of the detection system shown in Fig. 8.

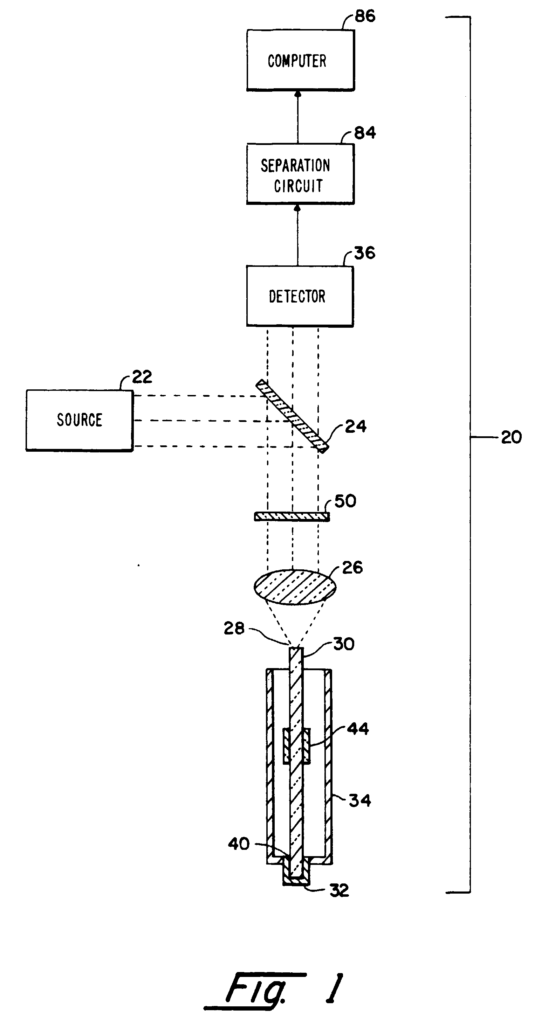

The present invention finds its utility in particular in a fiber-optic fluorescence immunoassay system for assaying a fluid, an exemplary embodiment of which system is identified at 20 in Fig. 1. System 20 comprises source 22 which provides a collimated beam of excitation light, beam splitting means such as dichroic mirror 24 arranged at a 45. angle relative to the path of a beam of the excitation light from source 22. System 20 further includes focusing means, such as objective lens 26, for gathering the collimated light reflected from dichroic mirror 24 and for focusing that light to form a cone, the apical end of which is directed into proximal end 28 of optical fiber 30. System 20 also includes mounting means 32 for supporting fiber 30 so that end 28 is in fixed, predetermined relation to, i.e., substantially at the focus of objective lens 26. Enclosure 34 is provided for receiving fiber 30 and for containing a solution to be assayed. Additionally, system 20 includes detector 36 for detecting fluorescence emitted from proximal end 28 of fiber 30 and transmitted through lens 26 and mirror 24, for providing output signals that vary as a function of the intensity of the emitted fluorescence.

Fiber 30 is an elongated body extending from proximal end or entrance face 28 to distal or terminal end 40. Fiber 30 preferably has a substantially circular cross-section dimensioned to be much smaller than the length of the fiber, although fibers of rectangular or other geometric cross-section may also be employed. The surface of proximal end 28 is typically a plane disposed normally to the longitudinal axis of the fiber, and is preferably highly polished to minimize any blemishes or surface defects that would tend to scatter incident and emitted light. Alternatively, proximal end 28 of the fiber may be configured in other desired optical shapes to serve, for example, as a magnifying or matching optical surface.

In a preferred embodiment, in which the fluorescence induced at the fiber surface by excitation radiation propagated along the fiber is collected or observed at the same proximal end of the fiber at which the excitation radiation is injected, it is desired to prevent stray radiation from going back up the fiber from distal end 40 to proximal end 28. Consequently, end 40 may be shaped to spill out light incident thereon internally, but preferably is coated with a material matching the index of refraction of the medium surrounding end 40, such material being both non-fluorescent and absorbent with respect to the excitation radiation. Typically, an epoxy resin loaded with carbon black serves such function.

Fiber 30 is adapted to propagate along its length, by multiple total internal reflection, optical excitation radiation entering proximal end 28 within a conical acceptance angle substantially symmetric with the long axis of the fiber and defined hereinbefore, as well known to those skilled in the fiber optics art. Fiber 30 may be any of a very large number of substantially homogeneous materials optically transparent to the excitation radiation, e.g., glassy materials such as glass; crystalline materials such as quartz, sapphire and the like; synthetic polymers such polyolefins, polystyrenes, polypropylenes and the like; and is preferably relatively stiff. However, inasmuch as the present invention is particularly adapted to permit the use of relatively low cost . synthetic polymer optical fibers, it is preferred that the optical fibers used with the present invention be made from synthetic, high-molecular weight polymers. Where fiber 30 is to be used in fluid assays as described hereinafter, the index of refraction ($n_0$) of the material forming fiber 30 must be satisfactorily greater than $n_1$, the index of refraction of the fluid being assayed. For purposes of an immunoassay apparatus, fiber 30 has a length ranging from 3cm to 5cm, with about 4cm being the preferred length. Fiber 30 typically has a diameter in the range of from about 0.5mm to 2mm, with about 1.5mm being the preferred diameter. It should be understood, however, that such length and diameter are merely exemplary and not limiting.

In an exemplary embodiment, it is intended that the operative portion or sensing zone of the fiber surface be defined by the dimensions of an activated region at which the assay is to be performed. To provide such activated region or sensing zone, that portion of the surface of the fiber is typically treated to provide coating 44 such as is described in detail in U.S. Patent No. 4,447,546. Coating 44 typically contains ligands immobilized on the surface of fiber 30, which ligands can form part of a complex that will fluoresce as a consequence of the assay, all as well known in the art. As described thus far, system 20 is virtually identical to the immunoassay system described in U.S. Patent No. 4,909,990.

For purposes of the present invention, assay system 20 also includes means for varying the numerical aperture at the evanescent zone adjacent the sensing portion of the surface of fiber 30 by alternately permitting only selected portions of the input cone to propagate along fiber 30. Typically, one such means is in the form of device 50 several embodiments of which are described in greater detail below.

As used herein, "surface binding fluorescence" refers to fluorescence generated by a complex, such as an immunoassay complex, bound to the active surface region or at the sensing zone of fiber 30. Such fluorescence is generated when a fluorescent moiety in the complex is excited by the evanescent wave component of the excitation radiation propagated along the fiber. As noted above, the surface binding fluorescence tunnels back into the fiber and is emitted from the proximal end of the fiber, such emitted fluorescence being referred to herein, inter alia, as "emission light" or "emission fluorescence".

As used herein the "low NA portion" of the excitation light (or the fluorescent emission as the case may be) refers to those light rays which extend perpendicular to or form a relatively large angle with the mean plane of proximal end 28 of fiber 30, e.g., rays substantially parallel to the longitudinal axis of the fiber. The "high NA portion" of the excitation light (or the fluorescent emission as the case may be) refers to those light rays extending at a relatively smaller angle with respect to the mean plane of the proximal end of the fiber. Thus, the low NA portion of the excitation light typically forms a relatively narrow-angle, solid cone of light rays, while the high NA portion of the excitation light may form a relatively wide-angle conical annulus of light rays, or may comprise the entire input cone.

Broad-band fluorescence generated in a polymeric substrate is a bulk rather than a surface phenomenon, and is unstable, exhibiting a photo-induced drift. This internally generated fluorescence is uniform in angle since the fluorophores radiate in random directions inside the fiber, whereas the surface binding fluorescence is highly non-uniform in angle, being heavily weighted in large angles as a result of the fluorescence tunneling back into the fiber. The desired fluorescent signals excited by the evanescent wave (and incorporating the data important to the assay) therefore scales as approximately $NA^9$ but the unwanted background fluorescence scales about as $NA^4$ ($NA^2$ for excitation, $NA^2$ for collection). As the total NA is restricted, the surface binding fluorescence will decrease more rapidly than the signal due to polymer fluorescence. The magnitude of the signal of the internally generated fluorescence at different values of numerical aperture at the sensing zone are directly correlatable with one another. One can simply discard the values measured for the fluorescence at low NA and consider the measured fluorescence at NA to be indicative of the desired fluorescent signal, but such an approach will provide inaccurate results inasmuch as the latter measurement still contains an unknown amount of the bulk fluorescence. Consequently, to obtain an accurate measure of the desired surface binding fluorescence signal, one can measure the fluorescence at two different NAs, and subtract from the value of the fluorescence at the higher NA, a function of the measure of the fluorescence at the lower NA.

A simplified approach in which, for conceptual understanding certain constant terms such as background fluorescence in the bulk solution under assay, dark level, reflected excitation and the like, are neglected, can be stated as follows:

Assume that two separate alternating series of measurements are taken, one at which the input excitation radiation to the fiber has been restricted or limited to only a small central core of the input cone, and therefore confers a comparatively low NA on the active zone of the fiber. The second series of measurement is taken when the excitation radiation includes all of the input cone and therefore establishes a relatively high NA at the active zone.

The peak amplitude of the total signal (T) detected when the active zone has a high NA ($NA_H$) is the sum of the binding fluorescent signal (D) that it is desired to find, and the undesired background fluorescence (U), or:

$$(3) \qquad T = U + D$$

The signals ($T_L$) detected when the sensing zone is excited at a low NA ($NA_L$), scale as about the fourth power of the NA for the undesired signal (U), but the desired signal scales as about the ninth power of the NA. To represent $T_L$ in terms of U and D, one can then provide an appropriate scale factor (K) as follows:

$$(4) \qquad K = (NA_H/NA_L)$$

Thus, $T_L$ can be represented as:

$$(5) \qquad T_L = U/K^4 + D/K^9$$

One can further assume, for ease in understanding, that the high NA signals (T) were taken as NA = 0.88 and the low NA signals ($T_L$) taken at NA = 0.4. Thus, K is (0.88/0.4) which, raised to the fourth power is about 23.4 and raised to the ninth power is about 1207, and equation (5) becomes:

$$(6) \qquad T_L = U/23.4 + D/1207$$

Rearranging the terms of equation (3) and substituting T - D for U in equation (6) one obtains:

$$(7) \qquad T_L = (T - D)/23.4 + D/1207$$

or

$$(8) \qquad D = 1.02T - 23.87T_L,$$

i.e., generally:

$$(9) \qquad D = C_1 T - C_2 T_L$$

where $C_1$ and $C_2$ are constants that are largely determined by the disk diameter. As will be apparent in the following description, both $T$ and $T_L$ are directly measurable using the principles of the present invention. The calculation required to obtain the desired signal (D) may be made in a conventional digital computer 86 coupled to receive the high NA and lower NA output signals from circuit 84. The specific programming steps required to accomplish the procedures for calculating the desired signal are not described herein being well within the ordinary skill in the art to write.

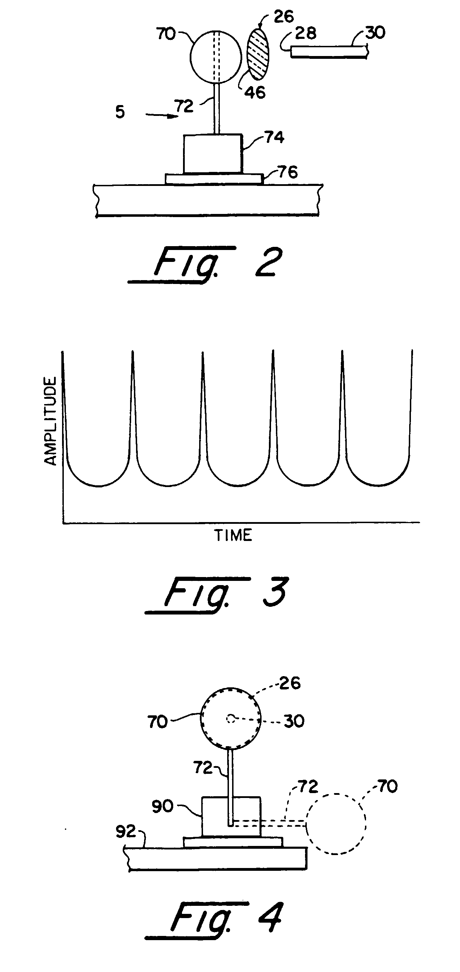

As shown in Fig. 2, a preferred embodiment of means for varying the NA of device 50, intended for use with fiber 30 having a circular proximal end 28, comprises circular, thin, flat disk 70 formed of material relatively opaque to the excitation radiation provided by source 22. Additionally, device 50 includes elongated shaft 72 coupled to one surface of disk 70 along a radius thereof, and motor 74 for driving shaft 72 in rotation about its longitudinal axis and disk 70 about a diameter thereof. Motor 74 is preferably positioned adjacent objective lens 26 so that shaft 72 extends perpendicularly to the optical axis of lens 26 and to the colinear axis of elongation of fiber 30. The length of shaft 72 and the placement of motor 74 are additionally selected so that disk 70 can be positioned adjacent, and parallel and centered with respect to front surface 46 of objective lens 26. Motor 74 is preferably supported on XYZ table 76 designed to cause the motor and hence disk 70 attached thereto, to be movably adjustable along three orthogonal axes, relative to proximal end 28 of fiber 30. By appropriate adjustment of table 76, importantly, disk 70 should be positioned as concentrically as possible with the optical axis of objective lens 26, so that, in this embodiment, when parallel to proximal end 28, disk 70 will permit passage of only a regular annulus (i.e., one having fixed values of inner and outer radii and a fixed width) formed from the outer portion of the input excitation radiation cone (or the output fluorescence emissions as the case may be).

Motor 74 serves to rotate shaft 72 and disk 70 attached thereto between a first or parallel position where the parallel flat surfaces of disk 70 extend parallel to the mean plane of proximal end 28 of fiber 30, and a second or perpendicular position where the flat surface of disk 70 extends perpendicular to the mean plane of proximal end 28. This rotation may be continuous in one direction (e.g., clockwise) or may be intermittent in alternating clockwise and counterclockwise directions between the parallel position and the perpendicular position. Preferably, motor 74 is positioned so that disk 70 is located as close as possible to the aperture plane of lens 26, given the constraint that the disk not contact the lens when in the perpendicular position. In one embodiment of the invention, motor 74 comprises a small DC motor so attached to shaft 72 as to cause the shaft to rotate continuously at a selected speed (e.g., in the range of 32 to 200 RPMs) about its longitudinal axis. In yet another embodiment, shaft 72 is driven by a stepping motor or relay that reversibly and intermittently moves disk 70 between its first and second positions.

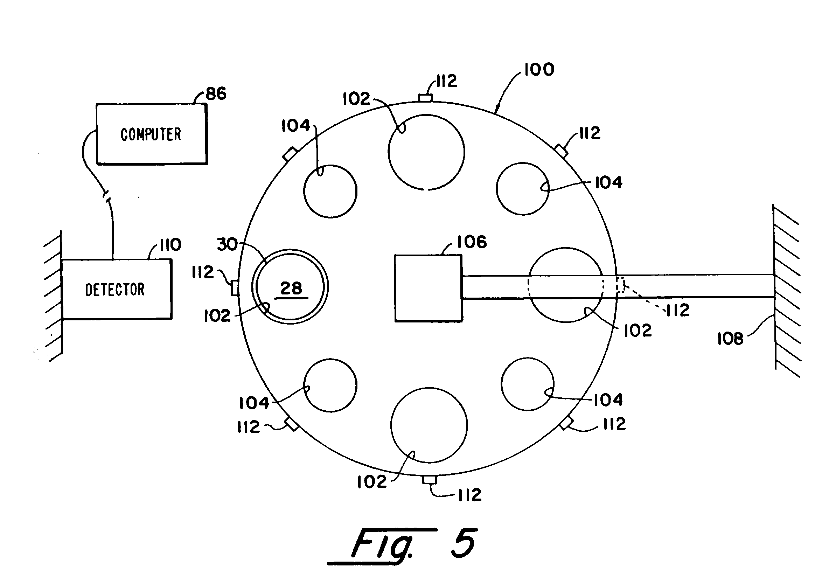

The diameter of disk 70 is selected so that when the latter is positioned parallel to proximal end 28, the disk will pass about its periphery an annular conical portion containing high NA excitation light and will block only the low NA portion of the excitation light (e.g., the solid internal conical portion of the cone of excitation light) from reaching fiber 30, and will also block or occlude the low NA portion of the fluorescence emissions (e.g., the solid internal conical portion of the fluorescence emissions) from reaching detector 36, but will permit the high NA portion of the fluorescence emissions to pass. Obviously, in this embodiment, when the disk is perpendicular to the mean plane of proximal end 28, substantially the entire input cone (and all of the output fluorescence emissions) is transmitted. The specific diameter of disk 70 will vary as a function of the size of objective lens 26, the size of the cone of excitation light as measured at disk 70, the relative amounts of low and high NA portions that one wishes to occlude and transmit, and other factors. The thickness of disk 70 is preferably selected so as to minimize the amount of excitation light it blocks when positioned to extend perpendicular to proximal end 28, as shown in Fig. 5. Typically, disk 70 has a thickness of about 0.1 mm.

The following description of the operation of the present invention relates to the preferred embodiment of means for varying the NA described above and illustrated in Fig. 2. Additionally, for the purpose of the following description, it is assumed fiber 30 is made of a polymer that will internally generate background fluorescence when exposed to the excitation light.

The portion of the fluorescence emitted from proximal end 28 of fiber 30 attributable to desired fluorescence generated as a consequence of surface binding of the constituents of the immunoassay complex, is determined based on information in the output signals of detector 36. If disk 70 is caused to rotate continuously between its perpendicular and parallel positions, the output signal from detector 36, shown in idealized form in Fig. 3, will modulate periodically between different values as a function of the rate of rotation of disk 70. Thus, when disk 70 is positioned parallel to proximal end 28, the magnitude (voltage) of the output signal of detector 36 will be relatively low because only the annular portion of the emission fluorescence can be observed around the disk, a substantial portion of the excitation light having been blocked from reaching fiber 30. That blocked portion of low NA light would have otherwise induced some fluorescence arising from the complex at the fiber surface and would have additionally excited fiber 30 to emit fluorescence at a relatively significant intensity level. When disk 70 is in the perpendicular position, the disk occludes relatively little of any of the excitation light or the resulting fluorescent emission. Consequently, the magnitude of the output signal of detector 36 is relatively large.

The output signal from detector 36, therefore, is essentially a modulated DC signal comprising a first component which primarily represents fluorescence arising responsively to excitation by the higher NA portion of the excitation light, and a second component which primarily represents fluorescence arising responsively to the low NA portion of the excitation light. The peak-to-peak modulation represents the low NA term and the envelope of the tops of the upper peaks represents the full NA term. The portion of the output signal excited by the high NA portion of the excitation light is not truly steady state because it will tend to vary with irregular and unpredictable changes in the fluorescence generated internally in the fiber. The periodic modulation component will, of course, be twice the frequency as that at which disk 70 is rotated by motor 74.

The difference between the RMS value and the peak-to-peak value of the modulation term is substantially a constant factor, and can be reflected in the value of the scaling factors used in equation (9). It is much easier electronically to obtain the RMS value of the modulation component and an average of the DC term rather than the peaks, and to this end, the present invention also includes any of a number of commercially available separating circuits 84 coupled to detector 36 for separating the output of the detector into an average value and an RMS equivalent value. In some cases, there may be significant feedthrough of the modulated signal in both the high NA and low NA channels. This feedthrough may be reduced by use of appropriate low pass filters on the output of the high and low NA channels.

Therefore, the output of circuit 84 is coupled to computer 86 in which electronic computations are made to essentially take the output of the detector and separate the average DC component from the modulation component, determine the RMS value of the latter, multiply it by a constant (i.e., amplify by a gain predetermined for the particular system) and subtract the result from the average DC component to obtain the desired output.

As means for varying the NA, several additional embodiments can be employed, one of which is illustrated in Fig. 4. This second embodiment of device 50 is similar to the embodiment illustrated in Fig. 2 in that it includes a shaft 72 attached to a surface of disk 70. However, the second embodiment of the invention differs from the embodiment shown in Fig. 2 in that it includes indexing motor 90 in place of motor 74, and disk 70 is mounted on shaft 72 in a common plane that is always parallel to proximal end 28. Indexing motor 90 is attached to fixed support 92 and is coupled to shaft 72. Motor 90 is designed and positioned to cause shaft 72 and disk 70 to move in that common plane perpendicularly to the axis of elongation of the fiber between a first position where the disk is not in the path of the excitation and emission light, as shown in phantom view in Fig. 4, and a second position where the disk is centered in the path of the excitation and emission light adjacent the front surface 46 of objective lens 26, as shown in solid view in Fig. 4. It will be appreciated that motor 90 can be designed to move disk 70 back and forth along the common plane, or in the alternative, in rotation in one direction within that common plane. In either event, it is clear that the motion of the shaft and disk will provide the requisite periodic changes in the NA of the sensing zone of an associated optical rod or fiber.

Fig. 5 illustrates a third embodiment of NA adjustment device 50 comprising opaque disk 100 having at least one relatively large and one relatively small circular aperture, and preferably a plurality of large circular apertures 102 and small circular apertures 104, positioned in alternate relation along a circular path adjacent the peripheral edge of disk 100 so that the center of each of the large and small apertures is positioned a predetermined radial distance from the center of the disk. The diameter of each large aperture 102 is selected so that when disk 100 is positioned adjacent objective lens 26 with a large aperture concentric with the optical axis of objective lens 26, both the high NA and low NA portions of the excitation and emission light will pass through the large aperture. The diameter of small aperture 104 is selected so that when the latter is similarly positioned relative to lens 26, the high NA portion of the excitation and emission light will be blocked by disk 100, and only the low NA light will pass through the small aperture.

The embodiment shown in Fig. 5 additionally comprises motor 106 for supporting disk 100 adjacent objective lens 26 and for causing the disk to rotate about its center. Motor 106 is attached to surface 108 and is sized and designed to support disk 100 adjacent front surface 46 of objective lens 26 so that the front and back surfaces of the disk are perpendicular to the optical axis of objective lens 26 and so that as the disk is rotated, apertures 102 and 104 will be successively moved into centered radiation with proximal end 28. Again, it is apparent that the motion of disk 100 and its associated apertures will provide the requisite periodic changes in the NA at the sensing zone of an associated optical rod or fiber.

Fig. 5 further preferably comprises a position detection system coupled to computer 86 for determining when apertures 102 and 104 are centered with respect to proximal end 28 of fiber 30 and for providing an output signal to the computer each time such centering occurs. A wide variety of apparatus may be used to provide notification to computer 86 of such alignment. However, an exemplary position detection system comprises auxiliary detector 110 secured to a fixed support adjacent the peripheral edge of disk 100. Detector 110 typically comprises means, such as a photodiode and associated optics (not shown), for transmitting an infrared radiation signal toward the peripheral edge of disk 100, and a plurality of reflectors 112 attached to the peripheral edge of disk 100 so that a reflector is positioned adjacent each of apertures 102 and 104. Detector 110 is designed to receive infrared radiation reflected from reflectors 112 and provide an output signal to computer 86 each time such reflection is received.

The desired signal (D) is determined using the embodiment of Fig. 5, in a manner similar to that heretofore described, except in two respects. First, the output signal from detector 36 must be gated so that the latter is provided to computer 86 only when detector 110 provides a signal to computer 86 indicating one of apertures 102 or 104 is centered with respect to proximal end 28 of fiber 30. Such gating may be accomplished using conventional gating circuits, as those of ordinary skill in the art will readily appreciate. Thus, the procedure for determining the desired signal described above is performed using information from detector 36 generated at the instant an aperture 102 or 104 is centered with respect to proximal end 28 of fiber 30. Second, the proportionality factor used in calculating the desired signal with the third embodiment of the invention is different from that used with the other embodiments of NA adjustment device 50. A different proportionality factor is required because small apertures 104 admit solely low NA light, while both high and low NA light can pass by disk 70 when in the "combined NA" position (i.e., when disk 70 is in the perpendicular position for the embodiment of Fig. 2, or when disk 70 is displaced to one side of fiber 30 for the embodiment of Fig. 4).

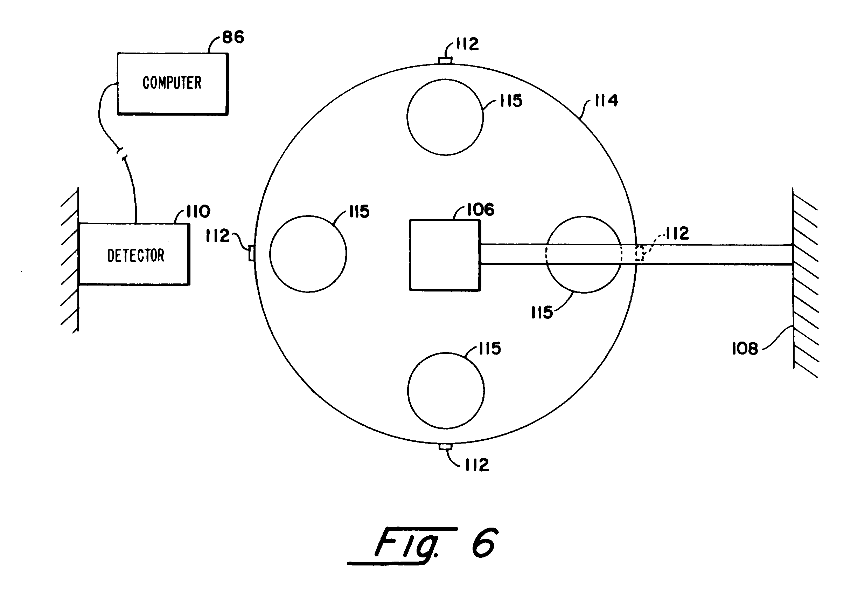

The embodiment of NA adjustment device 50 shown in Fig. 6 comprises clear disk 114 having a plurality of circular opaque spots 115 positioned a selected distance from one another along a circular line concentric with and adjacent the peripheral edge of the disk. The embodiment of Fig. 6 includes motor 106 mounted on surface 108 and coupled to and supporting disk 114 adjacent objective lens 26 so as to cause the disk to rotate so that spots 115 are successively moved into concentric relation with proximal end 28. Each of the plurality of spots 115 is sized so that when a spot is moved into concentric relation with proximal end 28, the spot will block a low NA portion of the excitation and emission light. All of the excitation light reaches proximal end 28 and all of the emission light passes through disk 114 when the clear portions of disk 114 between spots 115 are interposed between the source of excitation light and proximal end 28.

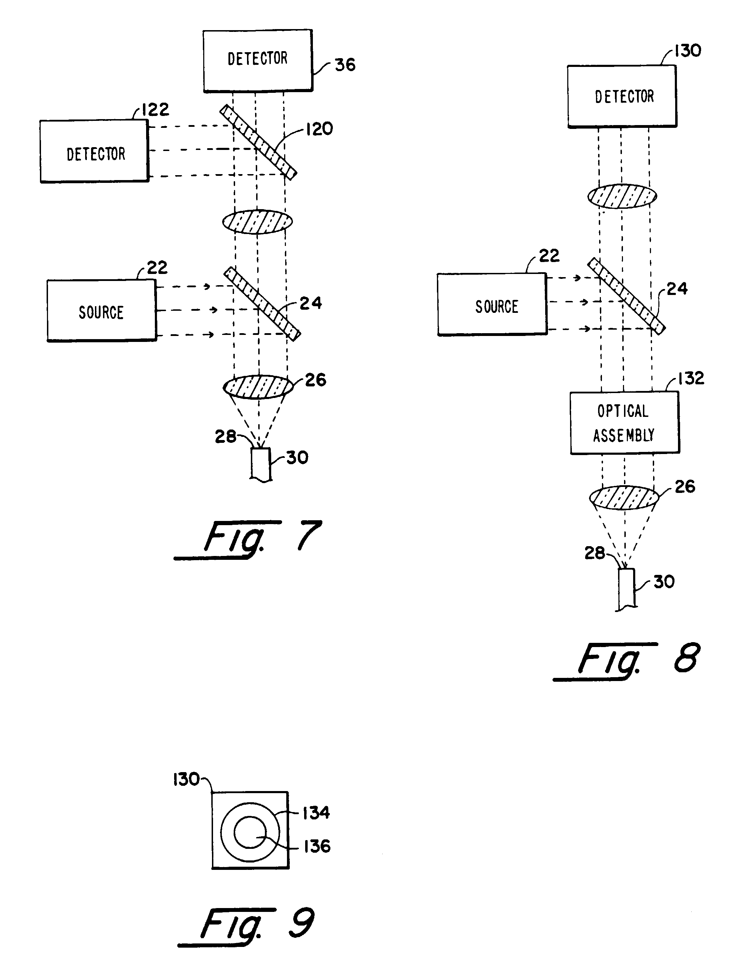

To avoid the need for circuit 84 for separating the output of detector 36 into an average DC signal and an RMS equivalent signal, the system illustrated in Fig. 1 may be modified, as shown in Fig. 7, to include elongated mirror 120 positioned at a 45. angle relative to the path of the light beam transmitted through mirror 24 toward detector 36. Mirror 120 is so shaped (e.g., typically as an oval) and dimensioned so as to occlude only the lower NA portion of the light, while transmitting the higher NA portion as an annulus around the sides of the mirror to detector 36. Second detector 122 for detecting fluorescence emissions from fiber 30 is positioned adjacent mirror 120 in the path of light reflected by the latter, and may be identical to detector 36.

By adding mirror 120 and detector 122 to the system of Fig. 1, the intensity of the low NA portion of the fluorescence emitted from fiber 30 may be determined based on the magnitude of the output signal of detector 122 and the intensity of the high NA portion of the fluorescence emitted from fiber 30 may be determined based on the magnitude of the output signal of detector 36. The desired signal (D) is then readily determined, so the relation of the latter to NA is approximately the square rather than the fourth power as heretofore described.

To achieve the advantages of the system illustrated in Fig. 7, without the need for two detectors, the system illustrated in Fig. 1 may be modified by eliminating circuit 84 and replacing detector 36 with detector 130, as illustrated in Figs. 8 and 9. Detector 130 includes first sensitivity region 134 for detecting the presence of the low NA light imaged along a conical path by optical assembly 132 and a second sensitivity region 136 for detecting the presence of the high NA light transmitted by optical assembly 132. Sensitivity regions 134 and 136 lie on a common plane. Detector 130 includes two output channels, one for each of sensitivity regions 134 and 136.

Optical assembly 132 typically comprises a lens system for gathering emission fluorescence at the aperture plane (i.e., that plane extending parallel to proximal end 28 of fiber 30 and positioned adjacent front surface 46 of objective lens 26) and focusing concentric separated images thereof onto detector 130. Detector 130 is positioned so that the plane on which its sensitivity regions 134 and 136 lie is coplanar with the focal plane on which the image at the aperture plane is focused by optical assembly 132.

## Claims

1. An assay system having an elongated optical element capable of propagating an input excitation radiation therethrough by total internal reflection and including adjacent a surface portion thereof, a sensing zone at which fluorescent moieties may be excited into an output fluorescent radiation by an evanescent wave generated from the propagating input excitation radiation, the assay system comprising a first means for directing the apical portion of a cone of said input excitation radiation into a first end of said element, characterised by;

    means for examining the angular distribution of the output fluorescent radiation excited by said evanescent wave of input excitation radiation and other output fluorescent radiation emitted from said first end of said element so that the amount of said output fluorescent radiation excited by said evanescent wave can be segregated from other radiation excited within the body of said element, said means for examining including means for controlling the egress of said output fluorescent radiation from said first end of said element so that only selected different portions of cones of said output fluorescent radiation that share the same axis of revolution as other cones of said output fluorescent radiation and have outer nappes that are conical, but not congruent with the nappe of said other cones of said output fluorescent radiation, including annuli thereof, are examined.

2. An assay system as claimed in claim 1, characterised in that said means for examining the angular distribution of said output fluorescent radiation excited by said input excitation radiation comprises means for varying the numerical apertures at said first end of said element.

3. An assay system as claimed in claim 1, characterised in that said means for examining the angular distribution of said output fluorescent radiation comprises means for detecting output fluorescence upon alternately controlling the numerical apertures of admission of said input excitation radiation into said first end of element so that only selected different conical colinear portions of said cone of said input excitation radiation will propagate along said element.

4. An assay system as claimed in claim 3, characterised in that said means for varying said numerical aperture comprises:

means for selecting from said cone of said input excitation radiation a first said portion which, when introduced into said first end of said element, provides a relatively high numerical aperture at said first end of said element;
means for selecting from said cone of said input excitation radiation a second said portion which, when introduced into said first end of said element, provides a relatively lower numerical aperture at said first end of said element; and
means for alternately introducing said portions of said cone of said input excitation radiation into said first end of said element for propagation along said element.

5. An assay system as claimed in claim 4, characterised in that said first portion is a conical annulus and said second portion is a cone having a solid angle of about the same value as the nappe of said annalus.

6. An assay system as claimed in claim 4, characterised in that said first portion is a conical annulus and said second portion is a cone having a solid angle of about the same value as the interior angle of said annulus.

7. An assay system as claimed in claim 3, characterised in that said means for varying said numerical aperture at said first end of said element comprises an opaque plate mounted for movement with respect to said cone between at least two positions, wherein said plate in the first of said positions constitutes said means for selecting a first portion of said cone, and in the second position constitutes said means for selecting a second portion of said cone.

8. An assay system as claimed in claim 7, characterised in that said plate is positioned between the source of said cone and said first end of said optical element concentrically with the latter, and is mounted for rotation about a shaft between said first position wherein said plate occludes at least a substantial portion of said cone and said second position wherein said plate occludes a relatively minor portion of said cone.

9. An assay system as claimed in claim 8, characterised in that said plate is substantially flat and is mounted for movement between said first and second positions substantially in its own plane.

10. An assay system as claimed in claim 8, characterised in that said plate is mounted for rotation in its own plane.

11. An assay system as claimed in claim 7, characterised in that said plate is substantially flat and relatively thin, and is mounted for movement substantially normal to its own plane between said first and second positions.

12. An assay system as claimed in claim 10, characterised in that said plate is mounted for rotation substantially normal to its own plane.

13. An assay system as claimed in claim 7, characterised in that said plate defines at least first and second apertures extending therethrough, said first aperture being dimensioned so as to constitute said means for selecting said first portion of said cone, and said second aperture being dimensioned so as to constitute said means for selecting said second portion of said cone.

14. An assay system for performing a fluorescence assay based upon the reaction between first and second constituents of a chemical complex, one of said constituents being immobilised on a surface portion of an elongated optical element capable of propagating an input excitation radiation therethrough by total internal reflection, and containing means excitable into an output fluorescence by an evanescent wave generated by propagation of said input excitation radiation, said fluorescence being used to identify said complex, said system characterised by providing, in combination:

means for directing said input excitation radiation substantially as an input cone into a first end of said element;
detector means for detecting said output fluorescence excited by said input excitation radiation propagating along said element and emitted from said first end of said element and for providing output signals which vary as a function of the intensity of said output fluorescence; and
means for controlling the egress of said output fluorescence from said first end of said element so that only selected different portions of cones of said output fluorescence that share the same axis of revolution as other output cones and have outer nappes that are conical, but not congruent with the nappe of said other output cones including annuli thereof, are examined.

15. An assay system as claimed in claim 14, characterised in that said means for directing said input excitation radiation comprises means for varying the numerical aperture of said input fluorescence.

16. An assay system as claimed in claim 15, characterised in that said means for varying numerical aperture comprises means for alternately permitting only selected different conical colinear portions of said input cone to propagate along said element and excite said output fluorescence.

17. An assay system as claimed in claim 16 characterised in that said means for varying said numerical aperture comprises:

means for selecting from said input cone a first conical colinear portion which, when introduced into said first end of said element, provides a relatively high numerical aperture at said surface portion;
means for selecting from said input cone a second conical colinear portion which, when introduced into said first end of said element, provides a relatively lower numerical aperture at said surface portion; and
means for alternately introducing said portions of said input cone into said first end of said element for propagation along said element.

18. An assay system as claimed in claim 17, characterised in that said system further comprises computer means coupled to said detector means for segregating, based on said input signals, the portion of said output fluorescence emitted from said first end of said element attributable to excitation of said element by said propagating input excitation radiation, from the portion of said output fluorescence attributable to the means excitable by said evanescent wave.

19. An assay system as claimed in claim 17, further comprising means for directing said output fluorescence substantially as an output cone of said output fluorescence toward said detector;

means for separating said output cone of said output fluorescence into two conical colinear portions; and
means for comparing said conical portions of said fluorescence with each other.

20. An assay system as defined in claim 19, characterised in that said detector means comprises:

mirror means for reflecting one of said two portions of said cone of fluorescence along an axis extending transversely to the axis of rotation of said cone of fluorescence;
first detector means for detecting said one of said portions of said cone of fluorescence and for providing first output signals which vary as a function of the intensity of the fluorescence detected thereby; and
second detector means for detecting the other of said portions of said cone of fluorescence and for providing second output signals which vary as a function of the intensity of the fluorescence detected thereby.

21. An assay system as claimed in claim 14, characterised in that said output fluorescence emitted from said first end of said element extends along a first axis and said detector means comprises:

optical means for imaging emission output fluorescence appearing at an aperture plane adjacent said first end of said element onto a focal plane; and
detector means having first and second sensitivity regions located at said focal plane, said first sensitivity region being positioned to detect a portion of output fluorescence emissions arising when the value of the numerical aperture is relatively high, said second sensitivity region being positioned to detect a portion of output fluorescence emissions arising when the value of the numerical aperture at a surface portion of said element is relatively low.

22. An assay system as claimed in claim 14, characterised in that said first end of said optical element comprises a planar entrance face, said fiber being made from a synthetic polymer.

23. An assay system as claimed in claim 22, further characterised in that said optical element comprises a first constituent of an immunoassay complex bonded to an active surface portion of said element.

**Patentansprüche**

1. Analysesystem mit einem langgestreckten optischen Element, das in der Lage ist, darin eine eingangsseitige Erregerstrahlung durch totale interne Reflexion zu verbreiten und das einen anliegenden Oberflächenabschnitt und eine empfindliche Zone aufweist, an der fluoreszierende Komponenten zu einer fluoreszierenden Ausgangsstrahlung anregbar sind durch eine schwindende Welle, die von der sich ausbreitenden eingangsseitigen Erregerstrahlung erzeugt ist, wobei das Analysesystem eine erste Einrichtung zum Richten des Spitzenabschnitts eines Konus der eingangsseitigen Erregerstrahlung in ein erstes Ende des Elementes aufweist, gekennzeichnet durch eine Einrichtung zum Prüfen der Winkelverteilung der fluorisierenden Ausgangsstrahlung, die von der schwindenden Welle der eingangsseitigen Erregerstrahlung angeregt wurde, sowie anderer fluoreszierende Ausgangsstrahlung, die vom ersten Ende des Elementes emittiert wird, so daß der Betrag der fluoreszierenden Ausgangsstrahlung, die von der schwindenden Welle angeregt wurde, von anderer Strahlung, die im Körper des Elementes erzeugt wurde, getrennt werden kann, wobei die Einrichtung zum Prüfen auch Mittel zum Überwachen des Austritts der fluoreszierenden Ausgangsstrahlung aus dem ersten Ende des Elementes einschließt, so daß nur ausgewählte Differenzanteile der Konen der fluoreszierenden Ausgangsstrahlung geprüft werden, die die gleiche Konusachse wie andere Konen der fluoreszierenden Ausgangsstrahlung sowie äußere Mäntel, die konisch, aber nicht kongruent mit den Mänteln der anderen Konen der fluoreszierenden Ausgangsstrahlung, einschließlich deren Ringe, haben.

2. Analysesystem nach Anspruch 1, dadurch gekennzeichnet, daß die Einrichtung zum Prüfen der Winkelverteilung der fluoreszierenden Ausgangsstrahlung, die durch eingangsseitige Erregerstrahlung erzeugt wurde, Mittel zum Verändern der numerischen Apertur am ersten Ende des Elementes aufweist.

3. Analysesystem nach Anspruch 1, dadurch gekennzeichnet, daß die Einrichtung zum Prüfen der Winkelverteilung der fluoreszierenden Ausgangsstrahlung Mittel zum Erfassen der Ausgangsfluoreszenz durch wechselweises Steuern der numerischen Aperturen des Eintritts der eingangsseitigen Erregerstrahlung in das erste Ende des Elementes aufweist, so daß nur ausgewählte konische, kolineare Differenzbereiche der Konen der eingangsseitigen Erregerstrahlung sich längs des Elementes verbreiten.

4. Analysesystem nach Anspruch 3, wobei die Mittel zum Verändern der numerischen Apertur gekennzeichnet sind durch:

   Mittel zum Abtrennen eines ersten Abschnitts vom Konus der eingangsseitigen Erregerstrahlung, wobei dieser abgetrennte Abschnitt, wenn er in das erste Ende des Elementes eintritt, eine relativ hohe numerische Apertur am ersten Ende des Elementes besitzt;

   Mittel zum Abtrennen eines zweiten Abschnitts der eingangsseitigen Erregerstrahlung, wobei dieser zweite Abschnitt, wenn er in das erste Ende des Elementes eintritt, eine relativ niedrige numerische Apertur am ersten Ende des Elementes aufweist; und

   Mittel zum wechselweisen Einführen der Abschnitte der Konen der eingangsseitigen Erregerstrahlung in das erste Ende des Elementes für die Verbreitung längs des Elementes.

5. Analysesystem nach Anspruch 4, dadurch gekennzeichnet, daß der erste Abschnitt ein konisches Ringrohr ist und daß der zweite Abschnitt ein Konus mit einem festen Konuswinkel ist, der etwa den gleichen Wert wie der Mantel des Ringrohrs aufweist.

6. Analysesystem nach Anspruch 4, dadurch gekennzeichnet, daß der erste Abschnitt ein konisches Ringrohr ist und der zweite Abschnitt ein Konus mit einem festen Konuswinkel, der etwa den gleichen Wert besitzt wie der innere Winkel des Ringrohrs.

7. Analysesystem nach Anspruch 3, dadurch gekennzeichnet, daß die Mittel zum Verändern der numerischen Apertur am ersten Ende des Elementes eine opake Platte aufweisen, die relativ zum Konus zwischen wenigstens zwei Positionen beweglich ist, wobei die Platte in einer ersten Position die Mittel zum Abtrennen eines ersten Teils des Konus und in der zweiten Position zum Abtrennen eines zweiten Teils des Konus bildet.

8. Analysesystem nach Anspruch 7, dadurch gekennzeichnet, daß die Platte zwischen der Quelle des Konus und dem ersten Ende des optischen Elementes konzentrisch zum Element positioniert ist sowie drehbar zwischen der ersten Position, bei der die Platte wenigstens einen wesentlichen Abschnitt des Konus abdeckt, und der zweiten Position, bei der die Platte einen verhältnismäßig kleinen Teil des Konus abdeckt, auf einer Welle angeordnet ist.

9. Analysesystem nach Anspruch 8, dadurch gekennzeichnet, daß die Platte im wesentlichen eben ist und im wesentlichen in ihrer eigenen Ebene zwischen der ersten und der zweiten Position bewegbar ist.

10. Analysesystem nach Anspruch 8, dadurch gekennzeichnet, daß die Platte in ihrer eigenen Ebene drehbar angeordnet ist.

11. Analysesystem nach Anspruch 7, dadurch gekennzeichnet, daß die Platte im wesentlichen eben und verhältnismäßig dünn ist sowie im wesentlichen normal zu ihrer Ebene zwischen der ersten und der zweiten Position beweglich ist.

12. Analysesystem nach Anspruch 10, dadurch gekennzeichnet, daß die Platte im wesentlichen normal zu ihrer Ebene drehbar ist.

13. Analysesystem nach Anspruch 7, dadurch gekennzeichnet, daß die Platte wenigstens erste und zweite Öffnungen aufweist, die sich durch die Platte erstrecken, wobei die erste Öffnung so dimensioniert ist, daß sie die Mittel zum Abtrennen des ersten Abschnitts des Konus bildet, und die zweite Öffnung so dimensioniert ist, daß sie die Mittel zum Abtrennen des zweiten Teils des Konus bildet.

14. Analysesystem zur Durchführung einer Fluoreszenzanalyse auf der Grundlage einer Reaktion zwischen ersten und zweiten Bestandteilen eines chemischen Komplexes, wobei der erste Bestandteil auf einem Oberflächenbereich eines langgestreckten optischen Elementes befestigt ist, welches in der Lage ist, eine eingangsseitige Erregerstrahlung durch totale interne Reflexion darin zu verbreiten, und wobei Mittel vorgesehen sind, um eine Ausgangsfluoreszenz zu erregen durch eine schwindende Welle, die durch Verbreitung der eingangsseitigen Erregerstrahlung erzeugt wurde, wobei die Fluoreszenz zum Identifizieren des Komplexes genutzt wird, gekennzeichnet durch die Kombination der folgenden Merkmale:

Mittel zum Richten der eingangsseitigen Eingangsstrahlung im wesentlichen als Eingangskonus in ein erstes Ende des Elementes;

Mittel zum Erfassen der Ausgangsfluoreszenz, die durch die längs des Elementes verbreitete, eingangsseitige Erregerstrahlung angeregt ist, und zum Erzeugen eines Ausgangssignals, das als Funktion der Intensität der Ausgangsfluoreszenz variiert; und

Mittel zum Steuern des Austritts der Ausgangsfluoreszenz aus dem ersten Ende des Elementes, so daß nur ausgewählte Differenzabschnitte der Konen der Ausgangsfluoreszenz geprüft werden, die die gleiche Konusachse wie andere Ausgangskonen sowie äußere Mäntel haben, die konisch, aber nicht kongruent mit den Mänteln der anderen Ausgangskonen, insbesondere deren Ringe, haben.

15. Analysesystem nach Anspruch 14, dadurch gekennzeichnet, daß die Mittel zum Richten der eingangsseitigen Erregerstrahlung Mittel zum Verändern der numerischen Apertur der Eingangsfluoreszenz aufweisen.

16. Analysesystem nach Anspruch 15, dadurch gekennzeichnet, daß die Mittel zum Variieren der numerischen Apertur Mittel aufweisen, die wechselweise nur ausgewählte konische kolineare Differenzabschnitte des Eingangskonus zur Verbreitung längs des Elementes und zur Erregung einer Ausgangsfluoreszenz durchlassen.

17. Analysesystem nach Anspruch 16, wobei die Mittel zum Variieren der numerischen Apertur bestehen aus:

Mittel zum Auswählen eines ersten konischen kolinearen Abschnitts aus dem Eingangskonus, wobei dieser Abschnitt beim Eintritt in das erste Ende des Elementes eine relativ hohe numerische Apertur am Oberflächenabschnitt besitzt;

Mittel zum Auswählen eines zweiten konischen kolinearen Abschnitts aus dem Eingangskonus, wobei dieser Abschnitt beim Eintritt in das erste Ende des Elementes eine relativ geringe numerische Apertur am Oberflächenabschnitt besitzt; und

Mittel zum wechselweisen Einführen dieser Abschnitte des Eingangskonus in das erste Ende des Elementes zur Verbreitung längs des Elementes.

18. Analysesystem nach Anspruch 17, dadurch gekennzeichnet, daß es ferner eine Computereinrichtung enthält, die mit der Einrichtung zum Erfassen gekuppelt ist, um auf der Basis der Eingangssignale den vom ersten Ende des Elementes emittierten Anteil der Ausgangsfluoreszenz, der auf die Erregung des Elementes durch Verbreitung der eingangsseitigen Erregerstrahlung zurückzuführen ist, von dem Anteil der Ausgangsfluoreszenz zu trennen, der auf die Erreger durch die schwindende Welle zurückzuführen ist.

19. Analysesystem nach Anspruch 17, ferner gekennzeichnet durch Mittel zum Richten der Ausgangsfluoreszenz im wesentlichen als Ausgangskonus der Ausgangsfluoreszenz auf den Detektor; Mittel zum Trennen des Ausgangskonus der Ausgangsfluoreszenz in zwei konische kolineare Abschnitte und Mittel zum gegenseitigen Vergleich der konischen Abschnitte der Fluoreszenz miteinander.

20. Analysesystem nach Anspruch 19, dadurch gekennzeichnet, daß die Mittel zum Erfassen bestehen aus:

einer Spiegeleinrichtung zum Reflektieren einer der beiden Konusabschnitte der Fluoreszenz längs einer Achse, die sich quer zur Rotationsachse des Fluoreszenzkonus erstreckt; eine erste Detektoreinrichtung zum Erfassen einer der Abschnitte des Fluoreszenzkonus und zum Erzeugen eines ersten Ausgangssignals, das als Funktion der Intensität dadurch erfaßten Fluoreszenz variiert; und

eine zweite Detektoreinrichtung zum Erfassen des anderen Abschnitts des Fluoreszenzkonus und zum Erzeugen eines zweiten Ausgangssignals, das als Funktion der Intensität der dadurch erfaßten Fluoreszenz variiert.

21. Analysesystem nach Anspruch 14, dadurch gekennzeichnet, daß die vom ersten Ende des Elementes erzeugte Ausgangsfluoreszenz sich längs einer ersten Achse erstreckt und daß die Einrichtung zum Erfassen besteht aus:

optischen Mitteln zum Abbilden der emittierten Ausgangsfluoreszenz, die an einer Aperturebene in der Nähe des ersten Endes des Elementes erscheint, auf eine Fokalebene; und

eine Detektoreinrichtung mit ersten und zweiten empfindlichen Bereichen, die sich an der Fokalebene befinden, wobei der erste empfindliche Bereich so angeordnet ist, daß er einen Abschnitt der emittierten Ausgangsfluoreszenz erfaßt, wenn der Wert der numerischen Apertur verhältnismäßig hoch ist, und wobei der zweite empfindliche Bereich so positioniert ist, daß er einen Abschnitt der emittierten Ausgangsfluoreszenz erfaßt, wenn der Wert der numerischen Apertur an der Oberfläche des Elementes verhältnismäßig gering ist.

22. Analysesystem nach Anspruch 14, dadurch gekennzeichnet, daß das erste Ende des optischen Elementes eine ebene Eingangsseite aufweist und aus synthetischem Polymer besteht.

23. Analysesystem nach Anspruch 22, dadurch gekennzeichnet, daß das optische Element einen ersten Bestandteil eines Immunprobenkomplexes aufweist, der mit einem aktiven Oberflächenbereich des Elementes verbunden ist.

## Revendications

1. Appareil d'analyse ayant un élément optique allongé susceptible de propager à travers lui par réflexion interne totale un rayonnement d'excitation d'entrée et comportant, adjacente à une portion de sa surface, une zone de détection au niveau de laquelle des fractions fluorescentes peuvent être excitées en un rayonnement fluorescent de sortie par une onde évanescente produite par le rayonnement d'excitation d'entrée se propageant, l'appareil d'analyse comprenant un premier moyen pour diriger la partie apicale d'un cône dudit rayonnement d'excitation d'entrée dans une première extrémité dudit élément, caractérisé par :

des moyens pour examiner la distribution angulaire du rayonnement fluorescent de sortie excité par ladite onde évanescente du rayonnement d'excitation d'entrée et d'un autre rayonnement fluorescent de sortie émis de ladite première extrémité dudit élément de telle sorte que la quantité dudit rayonnement fluorescent de sortie excité par ladite onde évanescente puisse être séparée de l'autre rayonnement excité à l'intérieur du corps dudit élément, lesdits moyens d'examen comprenant des moyens pour commander la sortie dudit rayonnement fluorescent de sortie de ladite première extrémité dudit élément de façon à examiner seulement des portions différentes sélectionnées de cônes dudit rayonnement fluorescent de sortie qui partagent le même axe de révolution que d'autres cônes dudit rayonnement fluorescent de sortie et qui ont des nappes extérieures qui sont coniques, mais non congruentes avec la nappe desdits autres cônes dudit rayonnement fluorescent de sortie, y compris leurs couronnes.

2. Appareil d'analyse selon la revendication 1, caractérisé en ce que lesdits moyens pour examiner la distribution angulaire dudit rayonnement fluorescent de sortie excité par ledit rayonnement d'excitation d'entrée comprennent des moyens pour faire varier les ouvertures numériques au niveau de ladite première extrémité dudit élément.

3. Appareil d'analyse selon la revendication 1, caractérisé en ce que lesdits moyens pour examiner la distribution angulaire dudit rayonnement fluorescent de sortie comprennent des moyens pour détecter une fluorescence de sortie en commandant alternativement les ouvertures numériques d'admission dudit rayonnement d'excitation d'entrée dans ladite première extrémité de l'élément de telle sorte que seules des portions colinéaires coniques différentes sélectionnées dudit cône dudit rayonnement d'excitation d'entrée se propageront le long dudit élément.

4. Appareil d'analyse selon la revendication 3, caractérisé en ce que lesdits moyens pour faire varier lesdites ouvertures numériques comprennent :

des moyens pour sélectionner sur ledit cône dudit rayonnement d'excitation d'entrée une première portion qui, lorsqu'elle est introduite dans ladite première extrémité dudit élément, procure une ouverture numérique relativement élevée au niveau de ladite première extrémité dudit élément;

des moyens pour sélectionner sur ledit cône dudit rayonnement d'excitation d'entrée une deuxième portion qui, lorsqu'elle est introduite dans ladite première extrémité dudit élément, procure une ouverture numérique relativement plus faible au niveau de ladite première extrémité dudit élément; et

des moyens pour introduire alternativement lesdites portions dudit cône dudit rayonnement d'excitation d'entrée dans ladite première extrémité dudit élément pour propagation le long dudit élément.

5. Appareil d'analyse selon la revendication 4, caractérisé en ce que ladite première portion est une couronne conique et ladite deuxième portion est un cône ayant un angle solide d'environ la même valeur que la nappe de ladite couronne.

6. Appareil d'analyse selon la revendication 4, caractérisé en ce que ladite première portion est une couronne conique et que ladite deuxième portion est un cône ayant un angle solide d'environ la même valeur que l'angle intérieur de ladite couronne.

7. Appareil d'analyse selon la revendication 3, caractérisé en ce que lesdits moyens pour faire varier ladite ouverture numérique au niveau de la première extrémité dudit élément comprennent une plaque opaque montée pour se déplacer par rapport audit cône entre au moins deux positions, ladite plaque dans la première desdites positions constituant lesdits moyens pour sélectionner une première portion dudit cône et, dans la deuxième position, constituant lesdits moyens pour sélectionner une deuxième portion dudit cône.

8. Appareil d'analyse selon la revendication 7, caractérisé en ce que ladite plaque est positionnée entre la source dudit cône et ladite première extrémité dudit élément optique, concentriquement par rapport à ce dernier, et est montée pour tourner autour d'un arbre entre ladite première position dans laquelle ladite plaque obture au moins une portion substantielle dudit cône et ladite deuxième position dans laquelle ladite plaque obture une portion relativement plus petite dudit cône.

9. Appareil d'analyse selon la revendication 8, caractérisé en ce que ladite plaque est pratiquement plate et est montée pour se déplacer entre ladite première position et ladite deuxième position pratiquement dans son propre plan.

10. Appareil d'analyse selon la revendication 8, caractérisé en ce que ladite plaque est montée pour tourner dans son propre plan.

11. Appareil d'analyse selon la revendication 7, caractérisé en ce que ladite plaque est pratiquement plate et relativement mince, et est montée pour se déplacer pratiquement perpendiculairement à son propre plan entre ladite première position et ladite deuxième position.

12. Appareil d'analyse selon la revendication 10, caractérisé en ce que ladite plaque est montée pour tourner pratiquement perpendiculairement à son propre plan.

13. Appareil d'analyse selon la revendication 7, caractérisé en ce que ladite plaque définit au moins des première et deuxième ouvertures s'étendant à travers elle, ladite première ouverture étant dimensionnée de façon à constituer lesdits moyens pour sélectionner ladite première portion dudit cône et ladite deuxième ouverture étant dimensionnée de façon à constituer lesdits moyens pour sélectionner ladite deuxième portion dudit cône.

14. Appareil d'analyse pour effectuer une analyse par fluorescence sur la base de la réaction entre des premier et deuxième constituants d'un complexe chimique, l'un desdits constituants étant immobilisé sur une portion de surface d'un élément optique allongé susceptible de propager à travers lui par réflexion interne totale un rayonnement d'excitation d'entrée, et contenant des moyens pouvant être excités en une fluorescence de sortie par une onde évanescente produite par la propagation dudit rayonnement d'excitation d'entrée, ladite fluorescence étant utilisée pour identifier ledit complexe, ledit appareil étant caractérisé en procurant, en combinaison :

des moyens pour diriger ledit rayonnement d'excitation d'entrée pratiquement sous forme d'un cône d'entrée dans une première extrémité dudit élément;

des moyens de détecteur pour détecter ladite fluorescence de sortie excitée par ledit rayonnement d'excitation d'entrée se propageant le long dudit élément et émise de ladite première extrémité dudit élément et pour procurer des signaux de sortie qui varient en fonction de l'intensité de ladite fluorescence de sortie; et

des moyens pour commander la sortie de ladite fluorescence de sortie de ladite première extrémité dudit élément de façon à examiner seulement des portions différentes sélectionnées de cônes de ladite fluorescence de sortie qui partagent le même axe de révolution que d'autres cônes de sortie et qui ont des nappes extérieures qui sont coniques, mais non congruentes avec la nappe desdits autres cônes de sortie, y compris leurs couronnes.

15. Appareil d'analyse selon la revendication 14, caractérisé en ce que lesdits moyens pour diriger ledit rayonnement d'excitation d'entrée comprennent des moyens pour faire varier l'ouverture numérique de ladite fluorescence d'entrée.

16. Appareil d'analyse selon la revendication 15, caractérisé en ce que lesdits moyens pour faire varier l'ouverture numérique comprennent des moyens pour permettre alternativement à certaines portions colinéaires coniques différentes sélectionnées dudit cône d'entrée de se propager le long dudit élément et d'exciter ladite fluorescence de sortie.

17. Appareil d'analyse selon la revendication 16, caractérisé en ce que lesdits moyens pour faire varier ladite ouverture numérique comprennent :

des moyens pour sélectionner sur ledit cône d'entrée une première portion colinéaire conique qui, lorsqu'elle est introduite dans ladite première extrémité dudit élément, procure une ouverture numérique relativement élevée *au* niveau de ladite portion de surface;

des moyens pour sélectionner sur ledit cône d'entrée une deuxième portion colinéaire conique qui, lorsqu'elle est introduite dans ladite première extrémité dudit élément, procure une ouverture numérique relativement plus faible au niveau de ladite portion de surface; et

des moyens pour introduire alternativement lesdites portions dudit cône d'entrée dans ladite première extrémité dudit élément pour propagation le long dudit élément.

18. Appareil d'analyse selon la revendication 17, caractérisé en ce que ledit appareil comprend en outre des moyens d'ordinateur couplés auxdits moyens de détecteur pour séparer, sur la base desdits signaux d'entrée, la portion de ladite fluorescence de sortie émise de ladite première extrémité dudit élément et pouvant être attribuée à l'excitation dudit élément par ledit rayonnement d'excitation d'entrée se propageant, de la portion de ladite fluorescence de sortie pouvant être attribuée aux moyens pouvant être excités par ladite onde évanescente.

19. Appareil d'analyse selon la revendication 17, comprenant en outre des moyens pour diriger ladite fluorescence de sortie pratiquement sous forme d'un cône de sortie de ladite fluorescence de sortie en direction dudit détecteur;

des moyens pour séparer ledit cône de sortie de ladite fluorescence de sortie en deux portions colinéaires coniques; et

des moyens pour comparer l'une avec l'autre lesdites portions coniques de ladite fluorescence.

20. Appareil d'analyse selon la revendication 19, caractérisé en ce que lesdits moyens de détecteur comprennent :

des moyens de miroir pour réfléchir l'une desdites deux portions dudit cône de fluorescence le long d'un axe s'étendant transversalement par rapport à l'axe de rotation dudit cône de fluorescence;

un premier moyen de détecteur pour détecter cette portion réfléchie dudit cône de fluorescence et pour procurer des premiers signaux de sortie qui varient en fonction de l'intensité de la fluorescence ainsi détectée; et

un deuxième moyen de détecteur pour détecter l'autre portion dudit cône de fluorescence et pour procurer des seconds signaux de sortie qui varient en fonction de l'intensité de la fluorescence ainsi détectée.

21. Appareil d'analyse selon la revendication 14, caractérisé en ce que ladite fluorescence de sortie émise de ladite première extrémité dudit élément s'étend le long d'un premier axe et en ce que les moyens de détecteur comprennent des moyens optiques pour former sur un plan focal une image de la fluorescence de sortie d'émission apparaissant au niveau d'un plan d'ouverture adjacent à ladite première extrémité dudit élément; et

   des moyens de détecteur ayant une première et une deuxième région de sensibilité situées au niveau dudit plan focal, ladite première région de sensibilité étant positionnée pour détecter une portion des émissions de fluorescence de sortie apparaissant lorsque la valeur de l'ouverture numérique est relativement élevée, ladite deuxième région de sensibilité étant positionnée pour détecter une portion des émissions de fluorescence de sortie apparaissant lorsque la valeur de l'ouverture numérique au niveau d'une portion de surface dudit élément est relativement faible.

22. Appareil d'analyse selon la revendication 14, caractérisé en ce que ladite première extrémité dudit élément optique comprend une phase d'entrée plane, ladite fibre étant faite d'un polymère synthétique.

23. Appareil d'analyse selon la revendication 22, caractérisé en outre en ce que ledit élément optique comprend un premier constituant d'un complexe d'essai immunologique lié à une portion de surface active dudit élément.

_Fig. 1_

_Fig. 2_

_Fig. 3_

_Fig. 4_

Fig. 5

EP 0 510 175 B1

Fig. 6

DETECTOR — 36

122 — DETECTOR

120

DETECTOR

22 — SOURCE

24

26

28

30

_Fig. 7_

130 — DETECTOR

22 — SOURCE

24

132 — OPTICAL ASSEMBLY

26

28

30

_Fig. 8_

130

134

136

_Fig. 9_